# EUROPÄISCHE PATENTSCHRIFT

(11) **EP 2 830 982 B1**
(45) Veröffentlichungstag und Bekanntmachung des Hinweises auf die Patenterteilung: **02.08.2017**
(21) Anmeldenummer: 13709902.4
(22) Anmeldetag: 15.03.2013
(51) Int. Cl.: B65H 63/06, G01N 33/36

(54) **VERFAHREN ZUR GARNÜBERWACHUNG**
YARN MONITORING METHOD
PROCÉDÉ DE SURVEILLANCE DE FIL

(30) Priorität: 26.03.2012 DE 102012102576
(43) Veröffentlichungstag der Anmeldung: 04.02.2015
(73) Patentinhaber: Maschinenfabrik Rieter AG, 8406 Winterthur (CH)
(72) Erfinder: BURCHERT, Mathias, 73760 Ostfildern (DE); JEHLE, Volker, 78337 Öhningen (DE)
(74) Vertreter: Bergmeier, Werner
(86) Internationale Anmeldenummer: PCT/EP2013/055334
(87) Internationale Veröffentlichungsnummer: WO 2013/143873

(56) Entgegenhaltungen:
- EP-A1- 0 877 108
- EP-A1- 1 712 507
- EP-A2- 0 995 711
- WO-A1-2011/038524
- DE-A1- 3 914 865
- DE-A1-102008 017 258

## Beschreibung

Die vorliegende Erfindung betrifft ein Verfahren zur Qualitätsüberwachung eines Garns an einer Textilmaschine, wobei das Garn eine Überwachungseinheit der Textilmaschine passiert, mit deren Hilfe zumindest eine von einem physikalischen Parameter des Garns abhängige Messgröße ermittelt wird, und wobei die Messgröße bzw. eine daraus abgeleitete Größe hinsichtlich ihrer Lage bezüglich wenigstens eines Referenzwerts ausgewertet wird. Darüber hinaus wird eine Textilmaschine vorgeschlagen, die wenigstens eine Überwachungseinheit zur Überwachung zumindest eines physikalischen Parameters des Garns und wenigstens eine mit der Überwachungseinheit in Wirkverbindung stehende Steuerung umfasst.

Im Stand der Technik ist es üblich, die Qualität eines Garns entweder unmittelbar nach seiner Herstellung (beispielsweise im Anschluss an die Spinnstelle einer Rotorspinnmaschine) oder aber während eines der Produktion nachfolgenden Handhabungsschritts (z. B. während des Umspulens von einer auf eine andere Garnspule) zu überwachen. Überwacht werden hierbei in der Regel physikalische Parameter bzw. von diesen Parametern abhängige Messwerte. Bekannt ist beispielsweise, die Garndicke oder die Haarigkeit des Garns durch Auswertung des von dem Garn auf eine Kontrollfläche geworfenen Schattens zu analysieren und daraus Rückschlüsse auf die Garnqualität zu ziehen.

Um aus den ermittelten Messwerten jedoch zuverlässige Aussagen über die Garnqualität treffen zu können, bedarf es stets einen Referenzwert, mit dem die ermittelten Messwerte verglichen werden können. Liegt die Abweichung der Messwerte vom Referenzwert innerhalb vordefinierter Grenzen, so geht man davon aus, dass die Qualität des Garns den Vorgaben entspricht. Eine gattungsgemäße Garnüberwachung zeigt beispielsweise die DE 10 2005 017 606 A1.

Der Referenzwert wird schließlich zu Beginn eines Produktionsvorgangs dadurch bestimmt, dass die zu überwachende Messgröße über einen bestimmten Zeitraum überwacht wird und in einen Referenzwert umgewandelt wird.

Nachteilig beim Stand der Technik ist jedoch die Tatsache, dass die anfängliche Referenzwertbestimmung zeit- und fehlerbehaftet ist. Insbesondere weicht die Qualität der gesamten Garncharge vom Soll ab, wenn die anfängliche Referenzwertbestimmung aus irgendeinem Grund fehlerhaft erfolgt. DE 39 14 865 A1 offenbart ein Verfahren zur Qualitätsüberwachung eines Garns an einer Textilmaschine gemäß dem Oberbegriff des Anspruchs 1. Aufgabe der vorliegenden Erfindung ist es daher, ein Verfahren zur Qualitätsüberwachung eines Garns an einer Textilmaschine vorzuschlagen, welches die genannten Nachteile nicht aufweist. Ferner soll eine Textilmaschine vorgeschlagen werden, die eine entsprechende Qualitätsüberwachung ermöglicht.

Die Aufgabe wird gelöst durch ein Verfahren sowie eine Textilmaschine mit den Merkmalen der unabhängigen Patentansprüche.

Das erfindungsgemäße Verfahren zur Qualitätsüberwachung eines Garns zeichnet sich nun dadurch aus, dass die Auswahl des bzw. der Referenzwert(e) unter Berücksichtigung einer oder mehrerer garnspezifischer und im Vorfeld der Auswertung definierter Kenngrößen des Garns erfolgt, wobei als Kenngröße die Garnhaarigkeit, das Material des Garns, die Garngleichmäßigkeit, die Faserfeinheit des Garns, die Steifigkeit des Garns, das Maß der Garndrehung und/oder die Garnspannung bei der Auswahl des Referenzwerts berücksichtigt wird. Der bzw. die Referenzwerte werden also nicht zu Beginn der Garnherstellung bzw. einer sonstigen Handhabung stets neu berechnet, sondern werden der Textilmaschine vorzugsweise bereits vor Beginn der eigentlichen Qualitätsüberwachung zur Verfügung gestellt.

Die Auswahl des passenden Referenzwerts erfolgt dabei auf Basis der genannten Kenngröße(n). Während die Kenngrößen, wie im Folgenden noch näher erläutert, beispielsweise physikalische Eigenschaften des herzustellenden Garns (z. B. dessen Haarigkeit) widerspiegeln können, ist es auch möglich, als Kenngröße einen garnspezifischen Code zu verwenden. Soll beispielsweise ein Baumwollgarn mit einer Garnnummer X, einer Drehung Y und einer Haarigkeit Z hergestellt werden, so weiß der Bediener (bzw. die Steuerung) der Textilmaschine, dass er (bzw. sie) den zugehörigen Referenzwert 123 auswählen und der Textilmaschine zur Verfügung stellen muss. Vor Beginn der Garnproduktion wird somit der entsprechende Referenzwert 123 in das Überwachungssystem eingelesen bzw. eingespielt. Im Verlauf der Produktion erfolgt schließlich der Vergleich der überwachten Messgröße (die beispielsweise die Garndicke widerspiegelt) mit dem genannten Referenzwert. Im Ergebnis ist auch nach einem Chargenwechsel immer wieder ein Garn mit reproduzierbaren Eigenschaften herstellbar, da für ein bestimmtes Garn (dass sich durch eine oder mehrere Kenngrößen definieren lässt), stets derselbe Referenzwert zur Verfügung steht bzw. ausgewählt wird.

Die Auswahl des Referenzwerts erfolgt entweder über die genannte garnspezifische Codenummer. Denkbar ist jedoch auch, dass mehrere Kenngrößen, wie z. B. die Garnnummer, die Haarigkeit oder auch das zu verspinnende Fasermaterial bei der Auswahl des Referenzwerts berücksichtigt wird.

Die Referenzwerte können schließlich entweder in eine zentrale Steuereinheit der Textilmaschine eingelesen werden, die schließlich die individuell ermittelten Messgrößen von den einzelnen Einheiten (z. B. Spinn- oder Spulstellen) erhält und einzeln mit dem Referenzwert vergleicht. Denkbar ist jedoch auch, dass die Referenzwerte an die einzelnen Überwachungseinheiten der jeweiligen Einheiten übermittelt werden und der Vergleich Referenzwert - Messgröße direkt an der jeweiligen Einheit erfolgt. Werden übermäßige Abweichungen der Messgröße von dem oder den (entsprechende Grenzwerte bildenden) Referenzwerten detektiert, so erfolgt entweder eine akustische und/oder optische Anzeige oder aber ein Eingriff in die Steuerung der jeweiligen Einheit, um die überwachte Messgröße bzw. den die Messgröße beeinflussenden physikalischen Parameter des Garns wieder in einen zulässigen Bereich zu regeln.

Besonders vorteilhaft ist es hierbei, wenn die jeweiligen Referenzwerte in einer Datenbank hinterlegt und bei Bedarf, d. h. vorzugsweise vor dem Beginn der Qualitätsüberwachung, aus dieser ausgelesen und an die Steuereinheit der Textilmaschine übermittelt werden (selbstverständlich kann die Datenbank auch Teil der Steuerung sein, so dass sich eine entsprechende Übermittlung erübrigen würde). Die Referenzwerte sind hierfür vorzugsweise mit einem oder mehreren der genannten Kenngrößen verknüpft, um die kenngrößenbasierte Auswahl des oder der passenden Referenzwerte zu ermöglichen bzw. zu vereinfachen. Als physikalischer Parameter wird die Garndicke berücksichtigt, wobei die Ermittlung der davon abhängigen Messgröße vorzugsweise mit Hilfe eines optischen Systems erfolgt, welches beispielsweise den vom Garn bei dessen Beleuchtung erzeugten Schatten hinsichtlich dessen Geometrie auswertet. Zum Einsatz kann beispielsweise eine Zeilenkamera kommen, welche die Pixel einer (oder mehrere nebeneinander liegende) Pixelreihe dahingehend auswertet, ob ein Schatten erkannt wird oder nicht. Die Auswertung liefert schließlich die Breite des durchlaufenden Garns (selbstverständlich vorausgesetzt, dass die Kamera so platziert ist, dass die Pixelreihen nicht parallel zur Garnlängsachse verlaufen). Das Garn passiert also eine (ein sichtbares oder nicht-sichtbares Licht erzeugende) Lichtquelle und erzeugt hierbei einen Schatten auf einer entsprechenden Kontrollfläche. Der Schatten, bzw. dessen räumliche Ausdehnung in eine oder mehrere definierte Richtungen wird mit Hilfe einer Kamera oder einem sonstigen System erfasst und in die Breite des Garns umgerechnet. Im Ergebnis stellt die Breite des Garns den genannten physikalischen Parameter dar, während die Messgröße beispielsweise die Stromstärke eines von der Kamera in Abhängigkeit der genannten Geometrie des Schattens erzeugten Messstroms sein kann. Denkbar ist auch, dass die Messgröße die Anzahl der Pixel darstellt, die einen Schatten detektieren. Als Kenngröße wird die Garnhaarigkeit, das Material des Garns, die Garngleichmäßigkeit, die Faserfeinheit des Garns, die Steifigkeit des Garns, das Maß der Garndrehung und/oder die Garnspannung bei der Auswahl des Referenzwerts berücksichtigt, so dass sich für ein bestimmtes Garn, dass von der Textilmaschine produziert oder anderweitig gehandhabt werden soll, ein oder mehrere zugehörige Referenzwerte aus der Datenbank auswählen lassen.

Vorteile bringt es zudem mit sich, wenn als Referenzwert ein Einzelwert ausgewählt wird, wobei der Referenzwert als Sollwert der Messgröße dient und der Betrag der Messgröße bzw. einer davon abgeleiteten Größe bezüglich seiner absoluten Abweichung vom Referenzwert ausgewertet wird. So könnten beispielsweise die positiven und negativen Abweichungen über die Zeit aufsummiert werden. Ein Alarm bzw. eine Anpassung ausgewählter Parameter der überwachten Einheit der Textilmaschine (beispielsweise die Rotordrehzahl an einer Spinnstelle einer Rotorspinnmaschine) erfolgt schließlich, wenn der über einen bestimmten Zeitraum aufsummierte Wert ober- bzw. unterhalb entsprechender Grenzwerte liegt.

Ebenso kann es von Vorteil sein, wenn der Referenzwert als Einzelwert vorliegt, und die Messgröße bzw. eine davon abgeleitete Größe dahingehend ausgewertet wird, ob ihr Betrag zwischen einem unterhalb des Referenzwerts liegenden Minimalwert und einem oberhalb des Referenzwerts liegenden Maximalwert liegt. Solange die Messgröße (bzw. deren Betrag) zwischen dem Minimal- und dem Maximalwert liegt, geht die zugehörige Steuerung davon aus, dass die Qualität des Garns den Vorgaben entspricht. Wird einer der Werte überschritten, so ist dies ein Zeichen, dass Material- und/oder Produktionsfehler vorliegen. Infolgedessen kann ein manueller oder auch automatischer Eingriff in die Steuerung zum Zwecke der Anpassung einzelner Betriebsparameter der Textilmaschine erfolgen. Denkbar ist auch die Abgabe eines entsprechenden Alarms, um einen Bediener auf die Abweichungen hinzuweisen.

Besondere Vorteile bringt es mit sich, wenn ein erster Referenzwert einen zulässigen Minimalwert und ein zweiter Referenzwert einen zulässigen Maximalwert definieren und im Rahmen der Qualitätsüberwachung überprüft wird, ob der Betrag der Messgröße bzw. einer davon abgeleiteten Größe zwischen dem Minimalwert und dem Maximalwert liegt. In diesem Fall beschränkt sich die Festlegung der Referenzwerte auf die Wahl zweier Grenzwerte, zwischen denen die ermittelte Messgröße liegen soll. Wenn beispielsweise bekannt ist, dass ein bestimmtes Garn (definiert durch die Kenngrößen Material, Festigkeit, Dehnbarkeit, etc.) eine Garndicke aufweisen soll, die zwischen A und B liegt, so werden die Beträge A und B als Referenzwerte für ein Garn mit den genannten Kenngrößen in der Datenbank hinterlegt. Soll nun dieses Garn produziert werden, so werden die genannten Referenzwerte aus der Datenbank ausgelesen und an die entsprechende Steuerung oder die einzelnen Überwachungseinheiten der Textilmaschine weitergeleitet.

Auch ist es von Vorteil, wenn die Ermittlung sowie die Auswertung der Messgröße kontinuierlich erfolgt. Unter kontinuierlich ist hierbei entweder eine Überwachung zu verstehen, die tatsächlich fortlaufend Messwerte erfasst, um ständig einen Echtzeitwert der genannten Messgröße zu erhalten. Alternativ ist es jedoch auch denkbar, dass die Ermittlung bzw. Auswertung in vorgegebenen Zeitabständen und damit ebenfalls kontinuierlich erfolgt. Ferner ist es möglich, mehrere Einzelmesswerte der Messgröße statistisch auszuwerten, so dass beispielsweise jede Minute ein Mittelwert der in der vorangegangenen Minute erfassten Messwerte gebildet und der Mittelwert mit dem bzw. den Referenzwert(en) verglichen wird.

Des Weiteren ist es vorteilhaft, wenn die Messgröße in Form von Absolutwerten ermittelt wird. Hierdurch können fehlerbedingende Abhängigkeiten von anderen Größen ausgeschlossen werden. Ein Vergleich der Messwerte zwischen einzelnen Garnchargen wird dadurch auf einfache Weise möglich. Hierdurch ist insbesondere sichergestellt, dass die Messgröße eines Garns XY auch dann noch aussagekräftig mit dem zugehörigen und in der Datenbank hinterlegten Referenzwert verglichen werden kann, wenn zwischen der Produktion zweier XY-Chargen Tage oder Wochen vergangen sind und in dieser Zeit ein Garn AB mit anderen Eigenschaften an der Textilmaschine produziert oder anderweitig gehandhabt wurde.

Ebenso ist es vorteilhaft, die Referenzwerte im Rahmen von Referenzmessungen ermittelt werden, wobei im Rahmen der Referenzmessungen jeweils der bzw. die vorher definierten Kenngröße(n) eines Referenzgarns sowie die von dem genannten physikalischen Parameter des Garns abhängige Messgröße des Referenzgarns ermittelt werden, wobei auf Basis der Messgröße bzw. einer davon abgeleiteten Größe ein oder mehrere Referenzwerte bestimmt werden, und wobei die Kenngröße(n) und der bzw. die Referenzwert(e) miteinander korreliert in der Datenbank hinterlegt werden. Mit anderen Worten werden die Daten der Datenbank dadurch generiert, dass die Messgröße während der Produktion eines in die Datenbank aufzunehmenden Garns während oder nach seiner Produktion ermittelt wird. Ebenso werden die nötigen Kenngrößen bestimmt, anhand derer der oder die Referenzwert(e) schließlich vor einem späteren Produktionsprozess eines dem Referenzgarn möglichst nahekommenden Garns aus der Datenbank ausgewählt werden. Die Referenzwerte werden schließlich in der Datenbank mit dem oder den relevanten Kenngrößen des Garns verknüpft, so dass zu einem beliebigen späteren Zeitpunkt der oder die Referenzwerte nach Eingabe der Kenngröße(n) von der Datenbank zur Verfügung gestellt werden können.

Besonders vorteilhaft ist es, wenn bei Überschreiten eines Maximalwerts oder Unterschreiten eines Minimalwerts des Referenzwerts die Qualität des Garns periodisch und vorzugsweise manuell überprüft wird und der oder die Referenzwert(e) in Abhängigkeit des Ergebnisses der Qualitätsprüfung neu festgelegt oder konstant gehalten wird. Mit anderen Worten müssen die in der Datenbank hinterlegten Referenzwerte nicht für alle Zeit konstant gehalten werden. So ist es beispielsweise denkbar, dass für ein Garn mit den Kenngrößen X, Y und Z ein Referenzwert A bzw. die Minimal- und Maximalwerte B und C hinterlegt sind. Unterschreitet nun die überwachte Messgröße den Minimalwert B, so ist dies zunächst ein Zeichen für die Steuerung und/oder den Bediener, dass die Qualität des Garns nicht den Vorgaben entspricht. Wird jedoch durch eine entsprechende manuelle oder mit Hilfe entsprechender Prüfvorrichtungen durchgeführten Kontrolle des Garns (an der Textilmaschine oder einem separaten Prüfort) festgestellt, dass die Qualität des Garns dennoch den Vorgaben entsprechen, die für ein Garn mit den Kenngrößen X, Y und Z festgelegt sind, so kann der Referenzwert A bzw. die Minimal- und Maximalwerte B und C in der Datenbank angepasst werden. Dies erfolgt beispielsweise dadurch, dass die genannten Werte derart verschoben werden, dass der (bezogen auf die ursprünglichen Werte außerhalb des zulässigen Bereichs liegende) Betrag der Messgröße wieder zwischen den Werten B und C liegt. Die Datenbank kann also fortlaufend angepasst werden, so dass ein einmalig festgelegter Referenzwert nicht bedeuten muss, dass spätere Anpassungen ausgeschlossen sind (beispielsweise kann der Abstand zwischen Minimal- und Maximalwert bei Bedarf auch verringert oder vergrößert werden, wenn beispielsweise der Abnehmer des Garns seine Qualitätsansprüche an das Garn verändert).

Besondere Vorteile bringt es mit sich, wenn vor der Überwachung des Garns der bzw. die mit der bzw. den Kenngröße(n) des Garns korrelierte(n) Referenzwert(e) aus der Datenbank entnommen und für die Qualitätsüberwachung herangezogen wird bzw. werden. Das Auslesen erfolgt also vorzugsweise immer dann, wenn entweder ein Wechsel des zu produzierenden bzw. des von der Textilmaschine zu handhabenden Garns erfolgen soll. Kommt es zu einem längeren Stillstand der Textilmaschine, so ist dann kein erneutes Auslesen des bzw. der Referenzwerte nötig, wenn das gleiche Garn produziert oder anderweitig gehandhabt (z. B. umgespult) werden soll und die Messgröße als absoluter Wert ermittelt wird.

Besondere Vorteile bringt es mit sich, wenn die Datenbank in einer Steuerung der Textilmaschine oder in der Überwachungseinheit hinterlegt ist. Alternativ ist es selbstverständlich auch möglich, die Datenbank fern der Textilmaschine abzulegen und die Daten bei einem Wechsel des zu produzierenden bzw. anderweitig zu handhabenden Garns manuell oder über entsprechende Netzwerkverbindungen an die Textilmaschine zu übertragen. In diesem Fall bestünde eine Datenbank für eine Vielzahl von Textilmaschinen, die an unterschiedlichen Produktionsstätten aufgestellt sein könnten.

Die erfindungsgemäße Textilmaschine zeichnet sich schließlich dadurch aus, dass sie eine Steuerung aufweist, die ausgebildet ist, die Garnqualität gemäß einem oder mehreren der vorangegangenen Ansprüche zu überwachen. Hinsichtlich der möglichen Varianten und Vorteile wird auf die bisherige und nachfolgende Beschreibung verwiesen. Insbesondere gilt dies für die bisherigen baulichen Merkmale der Textilmaschine (wie beispielsweise das bereits beschriebene optische System zur Messung der Geometrie des von dem zu überwachenden Garn bei Bestrahlung durch eine Lichtquelle erzeugten Schattens). Bei der Textilmaschine kann es sich generell um eine Maschine zur Produktion eines Garns (z. B. eine Rotor-, Luft- oder auch Ringspinnmaschine handeln). Ebenso kann die Textilmaschine dazu dienen, ein bereits produziertes Garn weiter zu bearbeiten oder anderweitig handzuhaben. Beispielsweise kann die Textilmaschine als Spulmaschine ausgebildet sein, mit deren Hilfe sich ein Garn von einer Spule auf eine andere umspulen lässt. Ferner sei an dieser Stelle darauf hingewiesen, dass die Textilmaschine eine einzige oder auch eine Vielzahl von Überwachungseinheiten besitzen kann, um entweder nur ein einziges oder aber eine Vielzahl von die jeweiligen Produktions- bzw. Handhabungseinheiten der Textilmaschine passierende bzw. verlassende Garne zu überwachen.

Weitere Vorteile der Erfindung sind in den nachfolgenden Ausführungsbeispielen beschrieben. Es zeigen:
- **Figur 1**: ein Schema zur Generierung eines Referenzwerts,
- **Figur 2**: ein Schema zur Auswahl eines Referenzwerts aus einer Datenbank,
- **Figur 3**: einen möglichen zeitlichen Verlauf einer Messgröße.

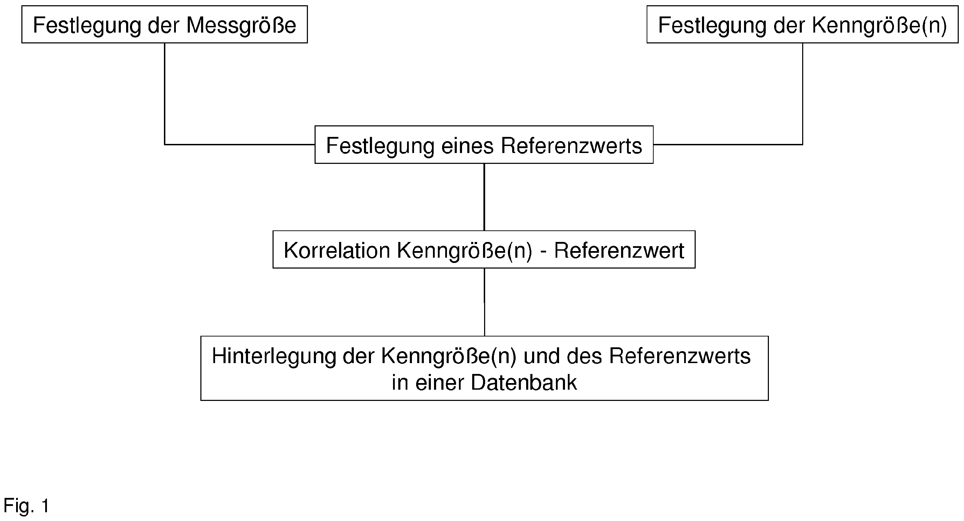

Figur 1 zeigt schematisch einen möglichen Ablauf zur Festlegung und Hinterlegung eines für die Durchführung des erfindungsgemäßen Verfahrens notwendigen Referenzwerts in eine Datenbank.

Zunächst wird eine Messgröße definiert, die bei der späteren Überwachung der Garnqualität an einer Textilmaschine erfasst und als Basis für die Beurteilung der Qualität herangezogen werden soll. Als Messgröße kann beispielsweise ein von einer Kamera erzeugtes Stromsignal sein, wobei die Kamera wiederum ausgebildet sein kann, die Geometrie, insbesondere die Breite, des Schattens zu detektieren, die das Garn bei einer Bestrahlung mit Licht erzeugt. In diesem Fall ist der Betrag der Messgröße also abhängig von der Breite (= räumliche Ausdehnung senkrecht zur Garnlängsachse) des Garns, d. h. die Breite des Garns wird überwacht.

Nach der Festlegung der Messgröße wird ein Referenzwert für die Messgröße definiert. Der Referenzwert stellt den Wert dar, den die Messgröße bei einwandfreier Qualität des Garns einnehmen sollte. Zusätzlich oder alternativ können selbstverständlich auch Referenzwerte in Form von zulässigen Minimal- und Maximalwerten definiert werden, zwischen denen die Messgröße bei einwandfreier Garnqualität liegen sollte.

Um nun die Referenzwerte immer dann aus einer die Referenzwerte enthaltenen Datenbank abrufen zu können, wenn ein bestimmtes Garn von der Textilmaschine hergestellt bzw. anderweitig gehandhabt werden soll, werden der bzw. die Referenzwerte nicht nur als Werte in der Datenbank hinterlegt. Vielmehr erfolgt die Verknüpfung mit einer oder mehreren garnspezifischen Kenngrößen, mit deren Hilfe sich die jeweiligen Referenzwerte stets einem bestimmten Garn zuordnen lassen. Als Kenngrößen können Codes (z. B. alphanumerische Codes) oder auch andere garnspezifische Kenngrößen, wie beispielsweise das Material des Garns, die Garndrehung, das längenbezogen Garngewicht, die Garnhaarigkeit, etc. herangezogen werden. Entscheidend ist lediglich, dass die einmal festgelegten Referenzwerte auch zu einem späteren Zeitpunkt noch eindeutig einem bestimmten Garn zuordenbar sind.

Mit anderen Worten ist es durch die erfindungsgemäße Lösung möglich, ein bestimmtes Garn (z. B. ein Baumwollgarn mit der Haarigkeit A, der Festigkeit B und der Garnnummer C) stets gleichwertig zu überwachen. So wird immer dann, wenn ein derartiges Garn überwacht werden soll, der zugehörige Referenzwert an die Steuerung der Textilmaschine bzw. den oder die entsprechenden Garnüberwachungseinheiten derselben weitergeleitet, so dass der Bezugspunkt der Überwachung stets derselbe ist. Erfolgt zusätzlich die Messung der Kenngröße in Form der Detektion von Absolutwerten, so muss die Überwachungseinheit nicht - wie im Stand der Technik üblich - vor jedem Wechsel des Garns neu kalibriert werden. Vielmehr kann das Garn stets von Beginn an zuverlässig, insbesondere reproduzierbar, hinsichtlich seiner Qualität überwacht werden.

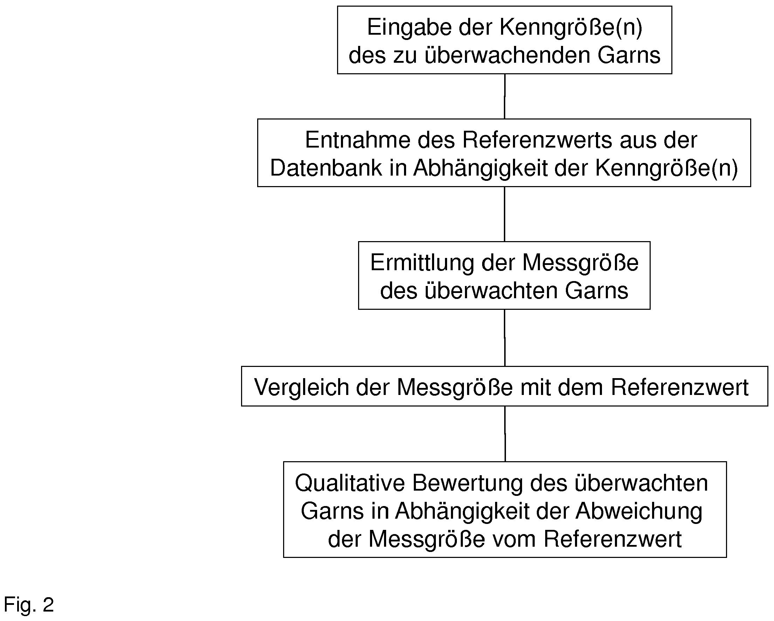

Eine mögliche Vorgehensweise bei der Auswahl des korrekten Referenzwerts bzw. entsprechender Minimal- und Maximalwerte zeigt Figur 2.

Zunächst müssen der oder die Kenngröße des zu überwachenden Garns in die Datenbank eingegeben werden. Die Datenbank liefert schließlich die gewünschten Werte, die mit der bzw. den Kenngrößen verknüpft sind. Der oder die Referenzwerte werden schließlich an die Steuerung der Textilmaschine bzw. entsprechende Überwachungseinheiten derselben übergeben und dienen als Grundlage der nun folgenden Qualitätsüberwachung.

Im Rahmen der Überwachung wird schließlich eine Messgröße des Garns durch vorzugsweise kontinuierliche Messung eines die Messgröße repräsentierenden Messwerts ermittelt und mit dem Referenzwert verglichen. Liegt die Messgröße innerhalb vorgegebener Grenzen (zulässiger Minimalwert, zulässiger Maximalwert), so ist dies gleichbedeutend mit einer akzeptablen Garnqualität. Unter- bzw. überschreitet die Messgröße die genannten zulässigen Werte, so entspricht die Garnqualität nicht mehr den Vorgaben. In diesem Fall wird entweder ein Alarm ausgelöst oder es erfolgt im Fall der Überwachung des Garns unmittelbar nach seiner Produktion ein Eingriff in die Steuerung der entsprechenden Produktionseinheit. Ziel kann es schließlich sein, die Produktionsparameter so zu regeln, dass die Messgröße stets innerhalb der genannten Grenzwerte liegt.

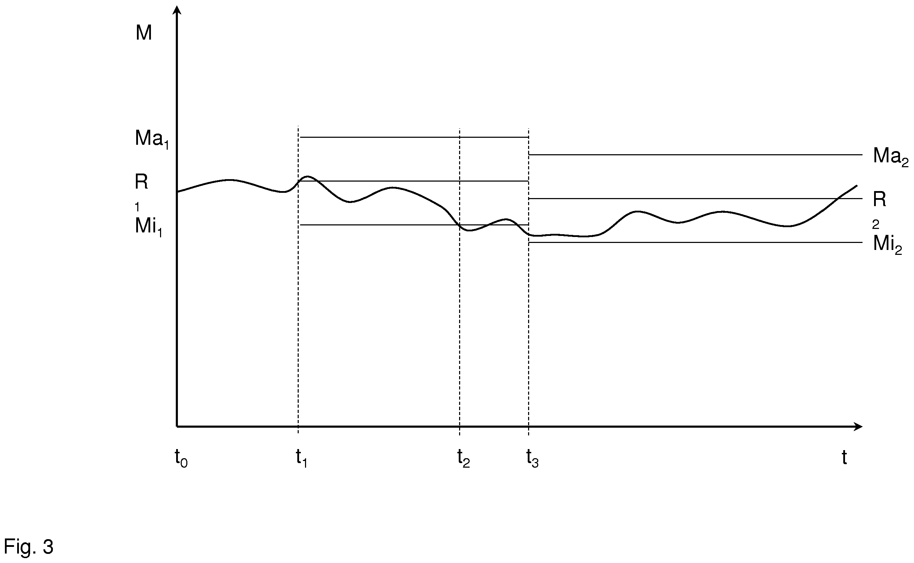

Eine Möglichkeit, den Referenzwert zu bestimmen bzw. anzugleichen, ist schließlich Figur 3 zu entnehmen, wobei M (y-Achse) für die Messgröße und t (x-Achse) für die Zeit steht.

Liegt für ein bestimmtes Garn noch kein Referenzwert R vor, so wird zunächst die Art der später zu überwachenden Messgröße M (beispielsweise das Signal einer Zeilenkamera, die die Breite des Garns überwacht) festgelegt. Anschließend wird die Erfassung der Messgröße M gestartet, während das entsprechende Garn die Überwachungseinheit passiert (t₀: Beginn der Erfassung der Messgröße M). Entspricht die Qualität des Garns den Vorgaben, so wird der im Zeitpunkt t₁ aktuell vorliegende Betrag der Messgröße M (bzw. ein davon abgeleiteter Wert) als Referenzwert R₁ definiert und - mit einer oder mehreren charakteristischen Kenngrößen (Haarigkeit, Garnnummer, Code des Garns und/oder weiterer ein bestimmtes Garn kennzeichnenden Größen) des Garns verknüpft - in der Datenbank abgelegt. Soll ein Garn mit vergleichbaren Eigenschaften zu einem späteren Zeitpunkt nochmals überwacht werden, so steht ein Referenzwert R₁ zur Verfügung, der jederzeit aus der Datenbank abrufbar ist.

Ergibt die Überwachung des Garns im weiteren zeitlichen Verlauf, in Figur 3 exemplarisch im Zeitpunkt t₂, dass die Messgröße M einen zulässigen Minimalwert Mi₁ unterschreitet (alternativ: einen zulässigen Maximalwert Ma₁ überschreitet), so wäre es möglich, dass die Garnqualität vom Bediener der Textilmaschine überprüft wird. Ergibt die Überprüfung, dass die Qualität noch immer den Vorgaben entspricht, so kann der Referenzwert R vom Wert R₁ auf den Wert R₂ korrigiert werden. Der korrigierte Wert wird schließlich zusammen mit einem entsprechend korrigierten Minimalwert Mi₂ und einem entsprechend korrigierten Maximalwert Ma₂ in der Datenbank hinterlegt (bzw. die alten Werte werden durch die korrigierten Werte überschrieben).

Im weiteren Verlauf dienen schließlich der neue Referenzwert R₂ bzw. die beiden Referenzwerte Ma₂ und Mi₂ als neue Bezugsgrößen, die für die Qualitätsüberwachung des Garns herangezogen werden können.

Die vorliegende Erfindung ist nicht auf das dargestellte und beschriebene Ausführungsbeispiel beschränkt. Abwandlungen im Rahmen der Patentansprüche sind ebenso möglich wie eine Kombination der Merkmale, auch wenn sie in unterschiedlichen Ausführungsbeispielen dargestellt und beschrieben sind.

### Bezugszeichenliste

- M: Messgröße
- t: Zeit
- R: Referenzwert
- Ma: zulässiger Maximalwert
- Mi: zulässiger Minimalwert

## Patentansprüche

1. Verfahren zur Qualitätsüberwachung eines Garns an einer Textilmaschine, wobei das Garn eine Überwachungseinheit der Textilmaschine passiert, mit deren Hilfe zumindest eine von einem physikalischen Parameter des Garns abhängige Messgröße (M) ermittelt wird, wobei als physikalischer Parameter die Garndicke berücksichtigt wird, wobei die Messgröße (M) bzw. eine daraus abgeleitete Größe hinsichtlich ihrer Lage bezüglich wenigstens eines Referenzwerts (R) ausgewertet wird, und wobei die Auswahl des bzw. der Referenzwert(e) (R) unter Berücksichtigung einer oder mehrerer garnspezifischer und im Vorfeld der Auswertung der Messgröße (M) definierter Kenngrößen des Garns erfolgt, **dadurch gekennzeichnet, dass** als Kenngröße die Garnhaarigkeit, das Material des Garns, die Garngleichmäßigkeit, die Faserfeinheit des Garns, die Steifigkeit des Garns, das Maß der Garndrehung und/oder die Garnspannung bei der Auswahl des Referenzwerts (R) berücksichtigt wird.

2. Verfahren gemäß Anspruch 1, **dadurch gekennzeichnet, dass** der bzw. die Referenzwert(e) (R) auf Basis der Kenngröße (e) aus einer, eine Vielzahl von Referenzwerten (R) umfassenden, Datenbank entnommen wird.

3. Verfahren gemäß einem oder mehreren der vorangegangenen Ansprüche, **dadurch gekennzeichnet, dass** die Ermittlung der von der Garndicke abhängigen Messgröße (M) mit Hilfe eines optischen Systems erfolgt, welches beispielsweise den vom Garn bei dessen Beleuchtung erzeugten Schatten hinsichtlich dessen Geometrie auswertet.

4. Verfahren gemäß einem oder mehreren der vorangegangenen Ansprüche, **dadurch gekennzeichnet, dass** als Referenzwert (R) ein Einzelwert ausgewählt wird, wobei der Referenzwert (R) als Sollwert der Messgröße (M) dient und der Betrag der Messgröße (M) bzw. einer davon abgeleiteten Größe bezüglich seiner absoluten Abweichung vom Referenzwert (R) ausgewertet wird.

5. Verfahren gemäß einem oder mehreren der Ansprüche 1 bis 5, **dadurch gekennzeichnet, dass** der Referenzwert (R) als Einzelwert vorliegt, und die Messgröße (M) bzw. eine davon abgeleitete Größe dahingehend ausgewertet wird, ob ihr Betrag zwischen einem unterhalb des Referenzwerts (R) liegenden Minimalwert (Mi) und einem oberhalb des Referenzwerts (R) liegenden Maximalwert (Ma) liegt.

6. Verfahren gemäß einem oder mehreren der Ansprüche 1 bis 4, **dadurch gekennzeichnet, dass** ein erster Referenzwert (R) einen zulässigen Minimalwert (Mi) und ein zweiter Referenzwert (R) einen zulässigen Maximalwert (Ma) definieren und im Rahmen der Qualitätsüberwachung überprüft wird, ob der Betrag der Messgröße (M) bzw. einer davon abgeleiteten Größe zwischen dem Minimalwert (Mi) und dem Maximalwert (Ma) liegt.

7. Verfahren gemäß einem oder mehreren der vorangegangenen Ansprüche, **dadurch gekennzeichnet, dass** die Ermittlung sowie die Auswertung der Messgröße (M) kontinuierlich erfolgt.

8. Verfahren gemäß einem oder mehreren der vorangegangenen Ansprüche, **dadurch gekennzeichnet, dass** die Messgröße (M) in Form von Absolutwerten ermittelt wird.

9. Verfahren gemäß einem oder mehreren der vorangegangenen Ansprüche, **dadurch gekennzeichnet, dass** die Referenzwerte (R) im Rahmen von Referenzmessungen ermittelt werden, wobei im Rahmen der Referenzmessungen jeweils der bzw. die vorher definierten Kenngröße(n) eines Referenzgarns sowie die von dem genannten physikalischen Parameter des Garns abhängige Messgröße (M) des Referenzgarns ermittelt werden, wobei auf Basis der Messgröße (M) bzw. einer davon abgeleiteten Größe ein oder mehrere Referenzwerte (R) bestimmt werden, und wobei die Kenngröße(n) und der bzw. die Referenzwert(e) (R) miteinander korreliert in der Datenbank hinterlegt werden.

10. Verfahren gemäß einem oder mehreren der vorangegangenen Ansprüche, **dadurch gekennzeichnet, dass** bei Überschreiten eines Maximalwerts (Ma) oder Unterschreiten eines Minimalwerts (Mi) der Referenzwerts (R) die Qualität des Garns periodisch und vorzugsweise manuell überprüft wird und der Referenzwert (R) in Abhängigkeit des Ergebnisses der Qualitätsprüfung neu festgelegt oder konstant gehalten wird.

11. Verfahren gemäß einem oder mehreren der vorangegangenen Ansprüche, **dadurch gekennzeichnet, dass** vor der Überwachung des Garns der bzw. die mit der bzw. den Kenngröße(n) des Garns korrelierte(n) Referenzwert(e) (R) aus der Datenbank entnommen und für die Qualitätsüberwachung herangezogen wird bzw. werden.

12. Verfahren gemäß einem oder mehreren der vorangegangenen Ansprüche, **dadurch gekennzeichnet, dass** die Datenbank in einer Steuerung der Textilmaschine oder in der Überwachungseinheit hinterlegt ist.

13. Textilmaschine mit wenigstens einer Überwachungseinheit zur Überwachung zumindest eines physikalischen Parameters eines Garns und wenigstens einer mit der Überwachungseinheit in Wirkverbindung stehenden Steuerung, **dadurch gekennzeichnet, dass** die Steuerung ausgebildet ist, die Garnqualität gemäß einem oder mehreren der vorangegangenen Ansprüche zu überwachen.

## Claims

1. Method for monitoring the quality of a yarn on a textile machine, wherein the yarn passes through a monitoring unit of the textile machine with whose assistance at least one measured variable (M) that depends on a physical parameter of the yarn is determined, wherein as a physical parameter the yarn thickness is considered, wherein the measured variable (M) or a variable derived from it is evaluated with regard to its position regarding at least one reference value (R), and wherein the reference value(s) (R) is/are selected taking one or several yarn-specific characteristic variables of the yarn into account, that has/have been defined before the evaluating of the measured variable (M), **characterized in that** yarn hairiness, yarn material, yarn uniformity, yarn fineness, yarn stiffness, extent of yarn twist and/or yarn tension are taken into account as a characteristic variable when selecting the reference value (R).

2. Method according to claim 1, **characterized in that** the reference value(s) (R) is/are taken from one database that includes multiple reference values (R) based on the characteristic variable(s).

3. Method according to one or several of the preceding claims, **characterized in that** the measured variable (M) depending on yarn thickness is determined with the help of an optical system that evaluates for example the shadow of the yarn that it casts when lit concerning its geometry.

4. Method according to one or several of the preceding claims, **characterized in that** an individual value is selected as reference value (R), in which case the reference value (R) serves as target value of the measured variable (M) and the magnitude of the measured variable (M) or of a variable derived from it is evaluated with regard to its absolute deviation from the reference value (R).

5. Method according to one or several of the claims 1 to 5, **characterized in that** the reference value (R) is available as individual value and the measured variable (M) or a variable derived from it is evaluated to find out whether its magnitude lies between a minimum value (Mi) that lies below the reference value (R) and a maximum value (Ma) that lies above the reference value (R).

6. Method according to one or several of the claims 1 to 4, **characterized in that** a first reference value (R) defines an admissible minimum value (Mi) and a second reference value (R) defines an admissible maximum value (Ma) and, as part of quality monitoring, it is checked whether the magnitude of the measured variable (M) or of a variable derived from it lies between the minimum value (Mi) and the maximum value (Ma).

7. Method according to one or several of the preceding claims, **characterized in that** the measured variable (M) is continuously being determined and evaluated.

8. Method according to one or several of the preceding claims, **characterized in that** the measured variable (M) is determined in form of absolute values.

9. Method according to one or several of the preceding claims, **characterized in that** the reference values (R) are determined as part of the reference measurements, wherein as part of the reference measurements, the previously defined characteristic variable(s) of a reference yarn and the measured variable (M) of the reference yarn that depends on the above-mentioned physical parameter of the yarn is/are determined in each case, wherein - based on the measured variable (M) or a variable derived from it - one or several reference values (R) are determined and wherein the characteristic variable(s) and the reference value(s) (R) are correlated with one another and stored in the database.

10. Method according to one or several of the preceding claims, **characterized in that** when the reference value (R) exceeds a maximum value (Ma) or falls below a minimum value (Mi), yarn quality is periodically checked, preferably manually, and the reference value (R) is reset or maintained constant depending on the result of the quality test.

11. Method according to one or several of the preceding claims, **characterized in that** before the yarn is monitored, the reference value(s) (R) correlated with the characteristic variable(s) of the yarn is/are taken from the database and used for quality monitoring.

12. Method according to one or several of the preceding claims, **characterized in that** the database is stored in a control of the textile machine or in the monitoring unit.

13. Textile machine with at least one monitoring unit for monitoring at least one physical parameter of a yarn and at least one control operationally connected to the monitoring unit, **characterized in that** the control is designed for monitoring yarn quality according to one or several of the preceding claims.

## Revendications

1. Procédé pour surveiller la qualité d'un fil dans une machine textile, dans lequel le fil parcourt une unité de surveillance de la machine textile, à l'aide de laquelle au moins une grandeur de mesure (M) dépendant d'un paramètre physique du fil est déterminée, dans lequel l'épaisseur du fil est prise en compte en tant que paramètre physique, dans lequel la grandeur de mesure (M) ou une grandeur dérivée de cette dernière est évaluée en ce qui concerne sa position par rapport à au moins une valeur de référence (R), et dans lequel la sélection de la ou des valeur(s) de référence (R) est effectuée compte tenu d'un ou de plusieurs paramètre(s) caractéristique(s) spécifique(s) au fil et défini(s) préalablement à l'évaluation de la grandeur de mesure (M), **caractérisé en ce que** sont pris en compte en tant que paramètre caractéristique lors de la sélection de la valeur de référence (R) le défibrage du fil, la matière du fil, la régularité du fil, la finesse du fil, la rigidité du fil, la mesure de la torsion du fil et/ou la tension du fil.

2. Procédé selon la revendication 1, **caractérisé en ce que** la ou les valeur(s) de référence (R) sont reprises depuis une base de données contenant une multitude de valeurs de référence (R) sur la base du paramètre caractéristique (e).

3. Procédé selon l'une ou plusieurs des revendications précédentes, **caractérisé en ce que** la détermination de la grandeur de mesure (M) dépendant de l'épaisseur de fil s'effectue à l'aide d'un système optique, lequel évalue par exemple l'ombre projetée par le fil lors de son illumination en ce qui concerne sa géométrie.

4. Procédé selon l'une ou plusieurs des revendications précédentes, **caractérisé en ce qu'**une valeur individuelle est sélectionnée en tant que valeur de référence (R), sachant que la valeur de référence (R) sert de valeur de cosigne de la grandeur de mesure (M) et le montant de la valeur de mesure (M) ou d'une grandeur dérivée de cette dernière est évalué en ce qui concerne son écart absolu par rapport à la valeur de référence (R).

5. Procédé selon l'une ou plusieurs des revendications 1 à 5, **caractérisé en ce que** la valeur de référence (R) est disponible en tant que valeur individuelle, et la grandeur de mesure (M) ou une grandeur dérivée de cette dernière est évaluée pour déterminer si son montant se situe entre une valeur minimale (Mi) située sous la valeur de référence (R) et une valeur maximale (Ma) située au-dessus de la valeur de référence (R).

6. Procédé selon l'une ou plusieurs des revendications 1 à 4, caractérisé en ce qu'une première valeur de référence (R) définit une valeur minimale admissible (Mi) et une seconde valeur de référence (R) définit une valeur maximale admissible (Ma) et, dans le cadre de la surveillance de la qualité, une vérification est effectuée pour déterminer si le montant de la grandeur de mesure (M) ou d'une grandeur dérivée de cette dernière est situé entre la valeur minimale (Mi) et la valeur maximale (Ma).

7. Procédé selon l'une ou plusieurs des revendications précédentes, **caractérisé en ce que** la détermination et l'évaluation de la grandeur de mesure (M) s'effectuent continuellement.

8. Procédé selon l'une ou plusieurs des revendications précédentes, **caractérisé en ce que** la grandeur de mesure (M) est déterminée sous forme de valeurs absolues.

9. Procédé selon l'une ou plusieurs des revendications précédentes, **caractérisé en ce que** les valeurs de référence (R) sont déterminées dans le cadre de mesures de référence, sachant que dans le cadre des mesures de référence, le ou les paramètre(s) caractéristique(s) d'un fil de référence défini(s) au préalable ainsi que la grandeur de mesure (M) du fil de référence dépendant du paramètre physique indiqué du fil sont respectivement déterminés, sachant qu'une ou plusieurs valeur(s) de référence (R) est/sont déterminées sur la base de la grandeur de mesure (M) ou d'une grandeur dérivée de cette dernière, et sachant que le(s) paramètre(s) physique(s) et la ou les valeur(s) de référence (R) sont stockés dans la base de données en corrélation les uns avec les autres.

10. Procédé selon l'une ou plusieurs des revendications précédentes, caractérisé en ce qu'en cas d'écart en plus par rapport à une valeur maximale (Ma) ou d'écart en moins par rapport à une valeur minimale (Mi) de la valeur de référence (R), la qualité du fil est contrôlée périodiquement et de préférence manuellement et la valeur de référence (R) est redéfinie ou maintenue constante en fonction du résultat du contrôle de qualité.

11. Procédé selon l'une ou plusieurs des revendications précédentes, caractérisé en ce qu'avant la surveillance du fil, la ou les valeur(s) de référence (R) corrélées avec le ou les paramètre(s) caractéristique(s) du fil est/sont prise(s) de la base de données et utilisée(s) pour la surveillance de la qualité.

12. Procédé selon l'une ou plusieurs des revendications précédentes, **caractérisé en ce que** la base de données est stockée dans une commande de la machine textile ou dans l'unité de surveillance.

13. Machine textile avec au moins une unité de surveillance pour la surveillance d'au moins un paramètre physique d'un fil et au moins une commande en liaison fonctionnelle avec l'unité de surveillance, **caractérisé en ce que** la commande se présente sous une forme pour surveiller la qualité du fil selon l'une ou plusieurs des revendications précédentes.
